# FASCICULE DE BREVET EUROPEEN

(11) **EP 0 827 512 B1**
(45) Date de publication et mention de la délivrance du brevet: **08.01.2003**
(21) Numéro de dépôt: 97915542.1
(22) Date de dépôt: 21.03.1997
(51) Int. Cl.: C07K 16/12, C12N 5/18, G01N 33/569

(54) **ANTICORPS SPECIFIQUE DE STAPHYLOCOCCUS AUREUS ET UTILISATIONS**
FÜR STAPHYLOCOCCUS AUREUS SPEZIFISCHER ANTIKÖRPER UND SEINE VERWENDUNG
ANTIBODY SPECIFIC FOR STAPHYLOCOCCUS AUREUS, AND USE THEREOF

(30) Priorité: 21.03.1996 FR 9603761
(43) Date de publication de la demande: 11.03.1998
(73) Titulaire: BIO MERIEUX, 69280 Marcy l'Etoile (FR)
(72) Inventeur: MERLIN, Sylviane, F-69300 Caluire (FR); BATTAIL, Nicole, F-69002 Lyon (FR); FLANDROIS, Jean-Pierre, F-69003 Lyon (FR); CARRET, Gérard, F-69230 Feyzin (FR)
(74) Mandataire: Guerre, Dominique
(86) Numéro de dépôt international: FR9700510
(87) Numéro de publication internationale: WO97034931

(56) Documents cités:
- EP-A- 0 323 331
- WO-A-90/15077
- FR-A- 2 679 923
- PATHOLOGIE BIOLOGIE 43 (5). 1995. 471-476, XP002020325 FELTEN A ET AL: "Review of slide agglutination tests, Pastorex Staph Plus, Slidex Staph-kit and Staph aureus, in the rapid detection of Staphylococcus aureus in clinic isolates."
- APMIS 101 (6). 1993. 487-491, XP000612057 CROIZE J ET AL: "IMPROVED IDENTIFICATION OF STAPHYLOCOCCUS - AUREUS USING A NEW AGGLUTINATION TEST RESULTS OF AN INTERNATIONAL STUDY."

## Description

La présente invention relève du domaine de la détection de bactéries *Staphylococcus aureus,* et notamment des souches de *Staphylococcus aureus* qui sont résistantes à la méticilline.

Par "détection", on entend collectivement toutes les techniques permettant d'identifier, qualitativement et/ou quantitativement, ou d'enrichir, purifier ou séparer un analyte biologique, en l'occurrence des bactéries *Staphylococcus aureus.*

Conformément au document EP-A-0 323 331, on connaît un réactif pour détecter des bactéries *Staphylococcus aureus*, y compris les souches résistantes à la méticilline, ce réactif comprenant :
- du fibrinogène,
- un anticorps reconnu par la protéine A de *Staphylococcus aureus,*
- un anticorps reconnaissant spécifiquement le sérotype capsulaire de type 5 de *Staphylococcus aureus* ou un anticorps reconnaissant spécifiquement le sérotype de type 8 de *Staphylococcus aureus,* et de préférence un mélange de ces deux anticorps.

Selon la présente invention, on apporte un anticorps, monoclonal ou polyclonal, qui reconnaît, de manière spécifique, un épitope commun aux souches de *Staphylococcus aureus* de différents sérotypes capsulaires, notamment les souches résistantes à la méticilline. Cet anticorps est choisi parmi les immunoglobulines de type G, les immunoglobulines de type M et les immunoglobulines de type A.

Une application particulièrement avantageuse de l'anticorps selon l'invention consiste à l'incorporer dans un réactif spécifique pour la détection de *Staphylococcus aureus,* y compris les souches résistantes à la méticilline. Par rapport au document EP-A-0 323 331, la sensibilité du réactif de l'invention est supérieure, car la capacité de reconnaissance des sérotypes capsulaires est plus importante.

Ainsi selon l'invention, on propose et décrit :
- un anticorps monoclonal ou polyclonal, spécifique d'un épitope commun aux souches de *Staphylococcus aureus* de différents sérotypes capsulaires, notamment des souches résistantes à la méticilline, ledit anticorps étant choisi parmi les immunoglobulines de type G, les immunoglobulines de type M et les immunoglobulines de type A ; cet anticorps reconnait à la fois spécifiquement au moins deux sérotypes capsulaires différents desdites souches de *Staphylococcus aureus ;* de préférence, il est choisi parmi les immunoglobulines de type G et les immunoglobulines de type M ;
- un anticorps monoclonal ou polyclonal tel que défini ci-dessus qui en particulier reconnait au moins le sérotype capsulaire de type 5 et le sérotype capsulaire de type 8 desdites souches de *Staphylococcus aureus,*
- un anticorps monoclonal susceptible d'être obtenu selon une technique adaptée de Galfe et al. (Nature, 266, 522-550, (1977)), en utilisant comme lignée cellulaire initiale pour la fusion, la lignée des myélomes SP2/0-Ag14 (ATCC CRL 1581) ; la fusion est effectuée avec des cellules de rate de souris de l'espèce BALB/C et de la souche BALB/CBYJICO (commercialisée par la Société IFFA Credo), immunisées par une souche de *Staphylococcus aureus* de type 5, selon les techniques classiques ; les clones obtenus ont été criblés par techniques immunoenzymatiques (ELISA) ; les clones sélectionnés ont été réinjectés par voie intrapéritonéale à des souris préparées au préalable pour la production de liquide d'ascite ; chaque anticorps monoclonal a été utilisé pour sensibiliser des particules de latex et a été testé pour sa spécificité à reconnaître les polyosides capsulaires de *Staphylococcus aureus ;* les anticorps monoclonaux dénommés respectivement P2G7A1E5 et P6D8D12E1 ont été choisis en raison de leur spécificité à reconnaître un épitope commun avec différents sérotypes capsulaires en particulier de type 5 et 8 ;
- un anticorps monoclonal susceptible d'être obtenu, ou tel qu'obtenu à partir de la lignée cellulaire hybridome déposée sous le N°96021514, le 15/02/1996 auprès de l'ECACC ;
- un anticorps monoclonal susceptible d'être obtenu, ou tel qu'obtenu à partir de la lignée cellulaire hybridome déposée sous le N°96021513, le 15/02/1996 auprès de l'ECACC ;
- une lignée cellulaire hybridome, telle que celle déposée sous le N°96021514, le 15/02/1996 auprès de l'ECACC, ou telle que celle déposée sous le N°96021513, le 15/02/1996 auprès de l'ECACC, ou toute autre lignée cellulaire hybridome dérivée, par exemple toute progéniture de cette lignée ; les lignées cellulaires sont revendiquées en tant que telles, ainsi que toute lignée cellulaire dérivée, c'est-à-dire susceptible de produire des anticorps présentant les mêmes caractéristiques immunologiques que celles décrites dans la présente invention ;
- un réactif spécifique de détection des bactéries *Staphylococcus aureus* et notamment des souches résistantes à la méticilline, comprenant au moins un anticorps reconnaissant spécifiquement au moins un épitope commun des différents sérotypes capsulaires des souches de *Staphylococcus aureus* résistantes à la méticilline, ledit réactif comprenant au moins un anticorps monoclonal ou polyclonal tel que défini précédemment ; ledit anticorps peut être fixé ou couplé, ou non, sur un support ou un marqueur ; par anticorps monoclonal ou polyclonal, on entend les anticorps tels que définis précédemment ainsi que tout anticorps présentant une spécificité croisée avec ceux-ci ;
- un réactif spécifique, notamment de détection des bactéries *Staphylococcus aureus* et notamment des souches résistantes à la méticilline, comprenant en outre du fibrinogène ou un composé à base de fibrinogène susceptible d'être reconnu par le facteur d'affinité pour le fibrinogène de *Staphylococcus aureus ;* le fibrinogène ou composé de fibrinogène peut être fixé ou couplé, ou non, sur un support ou sur un marqueur ;
- un réactif spécifique, notamment de détection des bactéries *Staphylococcus aureus* et notamment des souches résistantes à la méticilline, comprenant en outre des immunoglobulines ou leur fragment Fc reconnues par la protéine A de *Staphylococcus aureus,* fixées ou couplées, ou non, sur un support ou sur un marqueur,

Toutes sortes de support ou marqueur peuvent être envisagées selon l'invention.

On peut ainsi utiliser des particules en suspension. Ces particules sont notamment des particules de latex, telles que des billes de polystyrène ou des particules similaires, ayant de préférence une taille inférieure à 2 µm. A titre d'exemple, on peut citer les particules Estapor, commercialisées par la Société RHONE-POULENC, telles que :
- des particules de polystyrène K080, ayant un diamètre de 0,8 µm,
- des particules de polystyrène K109, ayant un diamètre de 0,8 µm,
- des particules de polystyrène ayant des groupes carboxyliques, PSI 480, ayant un diamètre de 0,8 µm.

On peut également utiliser des gels magnétiques, tels que les gels de polyacrylamide et/ou d'agarose contenant des particules magnétiques. On peut en outre utiliser des gels tels que Ultrogel et Magnogel (marques de commerce) de la Société IBF.

Le support utilisé dans un réactif selon la présente invention, peut être également des hématies par exemple de mouton.

Le support peut être sous la forme d'une plaque, d'un cône, d'une barrette, par exemple de polystyrène, ou d'un copolymère à base de styrène, d'un tube de verre ou analogue.

La fixation d'anticorps et de fibrinogène sur des particules en suspension, notamment de latex, peut être réalisée selon l'une des techniques suivantes :
- par adsorption passive, la fixation pouvant être spontanée, au cours d'une incubation des particules de latex dans une solution contenant les anticorps et le fibrinogène ; une incubation par exemple d'environ 2 heures à 20°C est souvent suffisante ;
- par covalence, la fixation pouvant être réalisée en créant une liaison covalente entre les anticorps et les groupements réactifs présents sur certaines particules du latex ; il est possible par exemple d'utiliser un carbodiimide pour créer la liaison covalente.

Lorsque le support est constitué d'hématies, celles-ci peuvent au préalable être sensibilisées par du fibrinogène ou toute autre molécule appropriée, selon les techniques classiques.

La concentration des anticorps à fixer sur les particules de latex, qui doit être déterminée pour chaque anticorps selon les méthodes connues, est habituellement comprise entre 0,1 µg et 100 µg de protéines anticorps par millilitre de latex en solution.

Dans un réactif selon l'invention, les anticorps et le fibrinogène peuvent être fixés sur une suspension unique de particules, par exemple de latex, ou bien être fixés sur des suspensions de particules respectivement différentes, telles que des hématies et des particules de latex, ou différents latex qui sont ensuite mélangés pour constituer le réactif.

Le marqueur peut être toute molécule biologique ou chimique, par exemple une enzyme, un haptène, un oligonucléotide, un élément radioactif...

D'autres objets de l'invention sont les suivants :
- un réactif tel que défini précédemment comprenant un anticorps de type immunoglobuline comprenant un fragment Fc susceptible de se fixer sur la protéine A de *Staphylococcus aureus* et/ou un autre composé à base de fibrinogène ou dérivé, susceptible de réagir avec le facteur d'affinité pour le fibrinogène desdites bactéries, ledit réactif comprenant de plus, au moins un anticorps polyclonal ou monoclonal susceptible de reconnaître spécifiquement différents sérotypes capsulaires de *Staphylococcus aureus,* notamment les sérotypes de type 5 et 8 ;
- un procédé pour détecter dans un échantillon biologique des bactéries *Staphylococcus aureus,* et notamment des souches résistantes à la méticilline, caractérisé en ce que :
   * on dispose d'un réactif, tel que précédemment défini,
   * on met en contact ledit échantillon avec ledit réactif,
   * on observe l'obtention d'une agglutination.

Les caractéristiques et avantages des objets de l'invention sont ci-après exposés et illustrés dans les Exemples 1-3.

### EXEMPLE 1 : Composition d'un réactif selon l'invention, appelé SLIDEX STAPH PLUS

Le réactif comprend un anticorps de type immunoglobuline comprenant un fragment Fc susceptible de se fixer sur la protéine A de *Staphylococcus aureus* et/ou un autre composé à base de fibrinogène ou dérivé, susceptible de réagir avec le facteur d'affinité pour le fibrinogène desdites bactéries, ledit réactif comprenant de plus, au moins un anticorps polyclonal ou monoclonal susceptible de reconnaître spécifiquement différents sérotypes capsulaires de *Staphylococcus aureus,* notamment les sérotypes de type 5 et 8.

### EXEMPLE 2 : Procédé d'obtention de l'anticorps contenu dans le réactif de l'Exemple 1

a) Préparation de cellules de rate de souris immunisées par une souche de *Staphylococcus aureus*
* Matériel :
Souches de *Staphylococcus aureus* CIP103313 de sérotype capsulaire 5 et CIP108314 de sérotype capsulaire 8.
Milieu de culture : gélose Columbia + 5 % de sang de mouton.
* Méthode :
Les souches conservées à - 80°C sont cultivées 2 fois sur gélose au sang de mouton.
Les colonies sont mises en suspensions en sérum physiologique à la concentration de 3 x 10⁹ germes/mL.
Une méthode d'inactivation a été utilisée : inactivation à 70°C pendant 1 heure.
Les vaccins sont préparés extemporanément.
* Origine de la culture cellulaire :
- Souche de *Staphylococcus aureus* de sérotype capsulaire 5
- Animal d'immunisation : souris femelles de l'espèce BALB/C et de la souche BALB/CBYJICO (fournies par IFFA Credo)
- Lignée myélomateuse SP2/0-AG14.

* Protocole d'immunisation :

| | | |
|---|---|---|
| J =0 | 3 x 10⁸ germes + ACF (1:1) | IP |
| J = 7,14 | 3 x 10⁸ germes | IP |
| J = 28,76 | 3 x 10⁸ germes | IP |
| J = 204 | Booster 3 x 10⁸ germes en NaCl 9 % | IV |
| J = 207 | fusion du 29/01/93, souris n°1 série 010 | |

* Culture en suspension
* Ploidie : hétéroploïde
* Conditions de culture :
- Milieu de base : Iscove's Modified Dulbecco Medium additionné de bicarbonate de sodium (3024 mg/l), de 10 % de sérum de veau foetal (SVF), pH 6,7 à 7,3, température 25°C.
- Réactifs additionnels : insuline (5 µg/l), 2-mercaptoéthanol (10 µM), éthanolamine (20 µM), pénicilline (100 U/ml), streptomycine (50 µg/ml).
- Technique de sub-culture : les cellules sont sub-cultivées tous les 2 ou 3 jours.

* Congélation des cellules :
- Composition du milieu : IMDM additionné de 10 % de SVF et 10 % de DMSO (diméthyl sulfoxyde - Sigma)
- Concentration cellulaire : 3,6 x 10⁶ cellules par ampoule (2 x 10⁶ cellules/ml)
- Méthode : congélation lente des cellules à -80°C dans du milieu IMDM - SVF 10 % - DMSO 10 %, 24h à 72h, puis conservation dans l'azote liquide -180°C.

b) Identification de l'hybridome stable
* Nombre de clonage effectués : 2
* Nom du clone : 6D8D12E1
* Isotypage : IgG2b,K
* Capacité de synthèse : taux d'Ig sécrétées dans le surnageant : ND.
* Spécificité antigénique :
   ELISA indirect (1)
   - plaques NUNC-maxisorb coatées avec 50 µL de germes (10⁹ g/ml) STAPH 5, séchés une nuit à 37°C,
   - saturer avec 100 µL de PBS-lait 1 % pendant 1 h à 37°C,
   - saturer avec 100 µL de PBS-sérum de cheval 1 %, incuber pendant 1 h à 37°C,
   - ajouter 100 µl de surnageant ou de liquide d'ascite dilués en PBS-Tween 20 0,05%, puis incubés 1 h à 37°C,
   - ajouter 100 µL d'Ig anti-Ig(H+L) de souris conjuguées à la PAL (Jackson Laboratories lot 24724) dilué au 1/2000 en PBS-BSA 1 % + sérum de cheval 1 %, 1 h à 37°C,
   - ajouter 100 µL de PNPP (BioMérieux réf. 60002990) à la concentration de 2 mg/ml dans de la DEA-HCL (Biomérieux réf. 60002989), pH9,8, incuber 30 min à 37°C,
   - bloquer la réaction avec 100 µL de NaOH 1 N.

   NB: 3 lavages sont effectués entre chaque étape avec 300 µL de Pbs-tween 20 0,05% et un lavage supplémentaire en eau distillée avant d'ajouter le PNPP.
   RESULTATS de (1) : cet anticorps est spécifique de *S. aureus* et ne reconnaît ni *S. Warneri,* ni *S. Hominis,* ni *S. Epidermidis,* ni *S. Cohnii*, ni *S. Haemolyticus.*
   Il reconnaît les souches *S. aureus* type 5, type 8, type B, type A, type MC31, et les souches non 5, non 8 (Ex: souches 1033, K8, 16N15).
   Technique d'agglutination directe (2)
   Dans des plaques à fond rond (Biomérieux M220 24A réf. 96350) mettre 50 µL de surnageants dilués en PBS + 50 µL de germes à 5 x 10⁸ g/ml ; agiter les plaques puis incuber à 22°C. La lecture est effectuée à l'oeil, 15 heures plus tard environ.
   RESULTATS de (2) : agglutination visible à l'oeil (4+).

c) Production d'anticorps in vivo
* Animal utilisé : souris femelles ayant de 4 - 6 semaines, de l'espèce BALB/C, de la souche BALB/CBYJICO (IFFA Credo).
* Préparation de l'inoculum : la suspension cellulaire est centrifugée à 200 g, 10 minutes, 25°C. Les cellules sont resuspendues dans du NaCl 9 % à raison de 10⁶ cellules/ml,
* injection de 10⁶ cellules/souris par voie intrapéritonéale, pour 20 souris,
* Temps de récolte du liquide d'ascite : 10 jours + 2 jours,
* Volume d'ascites : 245,5 ml,
* Volume du pool : 230,5 ml,
* Rendement : 93.9 %,

EXEMPLE 3 : Comparaison de la sensibilité et de la spécificité du réactif SLIDEX STAPH PLUS et de celles de divers réactifs commercialisés, dans la détection de différentes souches de *Staphylococcus aureus*
Le réactif SLIDEX STAPH PLUS a été comparé aux réactifs suivants :
- SLIDEX STAPH KIT: réf. 7 311 2 lot 674145 B (exp. 4/96), commercialisé par la Demanderesse,
- STAPHYSLIDE: réf. 5 508 1 lot 674775 A (exp. 9/96), commercialisé par la Demanderesse,
- PASTOREX STAPH PLUS commercialisé par Diagnostic Pasteur : réf. 56356 lot 4LO42R (exp 2/96) et lot SE 047 R (exp. 7/96),
- STAPHAUREX PLUS commercialisé par Murex : Réf ZL 33 lot K61691 0 (exp. 1 1/95) et lot K9251 1 0 (exp. 8/96).

Pour déterminer la sensibilité de chacun de ces réactifs, les 346 souches de *Staphylococcus aureus* suivantes ont été testées :
- 118 souches *S. aureus* résistantes à la méticilline et n'ayant pas le facteur d'affinité pour le fibrinogène (Clumping Factor négatif) (origine : France, Allemagne, Angleterre, Benelux, Italie, Espagne, USA, USA Kansas)
- 131 souches *S. aureus* résistantes à la méticilline et ayant le facteur d'affinité pour le fibrinogène (Clumping Factor positif) (origine : France, Allemagne, Angleterre, Benelux, Italie, Espagne, USA, Australie, Hong-Kong)
- 97 souches *S. aureus* sensibles à la méticilline (origine : France, Angleterre, Benelux, Italie, Espagne, Pologne, Australie, Hong Kong)

En outre, pour déterminer la spécificité de chacun de ces réactifs, 155 souches *Staphylococcus* non aureus ont été testées (origine : France, Allemagne, Angleterre, Benelux, Italie, Espagne, Pologne, USA, Australie, Hong-Kong).
Les souches ont été cultivées sur gélose Columbia + 5 % de sang de mouton.
a) Résultats de sensibilité obtenus avec les 118 souches *S. aureus* résistantes à la méticilline et n'ayant pas le facteur d'affinité pour le fibrinogène
Les résultats sont rassemblés dans le Tableau 1 ci-dessous :

**Tableau 1**

| Nombre de souches | Slidex Staph Plus | Slidex Staph Kit | Pastorex Staph Plus | Staphaurex Plus |
|---|---|---|---|---|
| 76 | + | + | + | + |
| 1 | + | + | + | inint. |
| 1 | + | + | inint. | + |
| 1 | + | + | - | + |
| 9 | + | + | + | - |
| 4 | + | + | - | - |
| 18 | + | - | + | + |
| 1 | + | - | + | - |
| 5 | + | - | - | - |
| 1 | - | + | + | + |
| 1 | - | + | - | - |
| total : 118 | 116 | 94 | 106 | 97 |
| Sensibilité | 98.30 % | 79.67 % | 90.6 % | 82.9 % |

inint signifie "ininterprétable".
Conformément au Tableau 1, SLIDEX STAPH PLUS a montré une sensibilité significativement supérieure (98,30 %) à celle de SLIDEX STAPH KIT (79.67 %), de PASTOREX *STAPH* PLUS (90,6 %) et STAPHAUREX PLUS (82,9 %).
b) Résultats obtenus avec les 131 souches *S. aureus* résistantes à la méticilline et ayant le facteur d'affinité pour le fibrinogène
Les résultats sont rassemblés dans le Tableau 2 ci-dessous :

**Tableau 2**

| Nombre de souches | Slidex Staph Plus | Slidex Staph Kit | Pastorex Staph Plus | Staphaurex Plus |
|---|---|---|---|---|
| 123 | + | + | + | + |
| 2 | + | + | + | inint. |
| 4 | + | + | + | - |
| 2 | +* | + | +* | - |
| total : 131 | 131 | 131 | 131 | 123 |
| Sensibilité | 100% | 100% | 100% | 95.35 % |

| | | | | |
|---|---|---|---|---|
| * agglutination lente inint signifie "ininterprétable". | | | | |

c) Résultats obtenus avec les 97 souches *S. aureus* sensibles à la méticilline
Les résultats sont rassemblés dans le Tableau 3 ci-dessous :

**Tableau 3**

| Nombre de souches | Slidex Staph Plus | Slidex Staph Kit | Staphyslide | Pastorex Staph Plus | Staphaurex Plus |
|---|---|---|---|---|---|
| 94 | + | + | + | + | + |
| 1 | + | inint. | + | + | + |
| 2 | + | inint. | inint. | + | + |
| total : 97 | 97 | 94 | 95 | 97 | 97 |
| Sensibilité | 100% | 100 % | 100% | 100% | 100% |

inint signifie "ininterprétable".
Conformément à ce tableau, l'ensemble des souches a été détecté, ce qui donne une sensibilité de 100 % pour chacun des réactifs.
d) Résultats obtenus avec les 155 souches *S. non aureus*
Les résultats sont rassemblés dans le Tableau 4 ci-dessous : 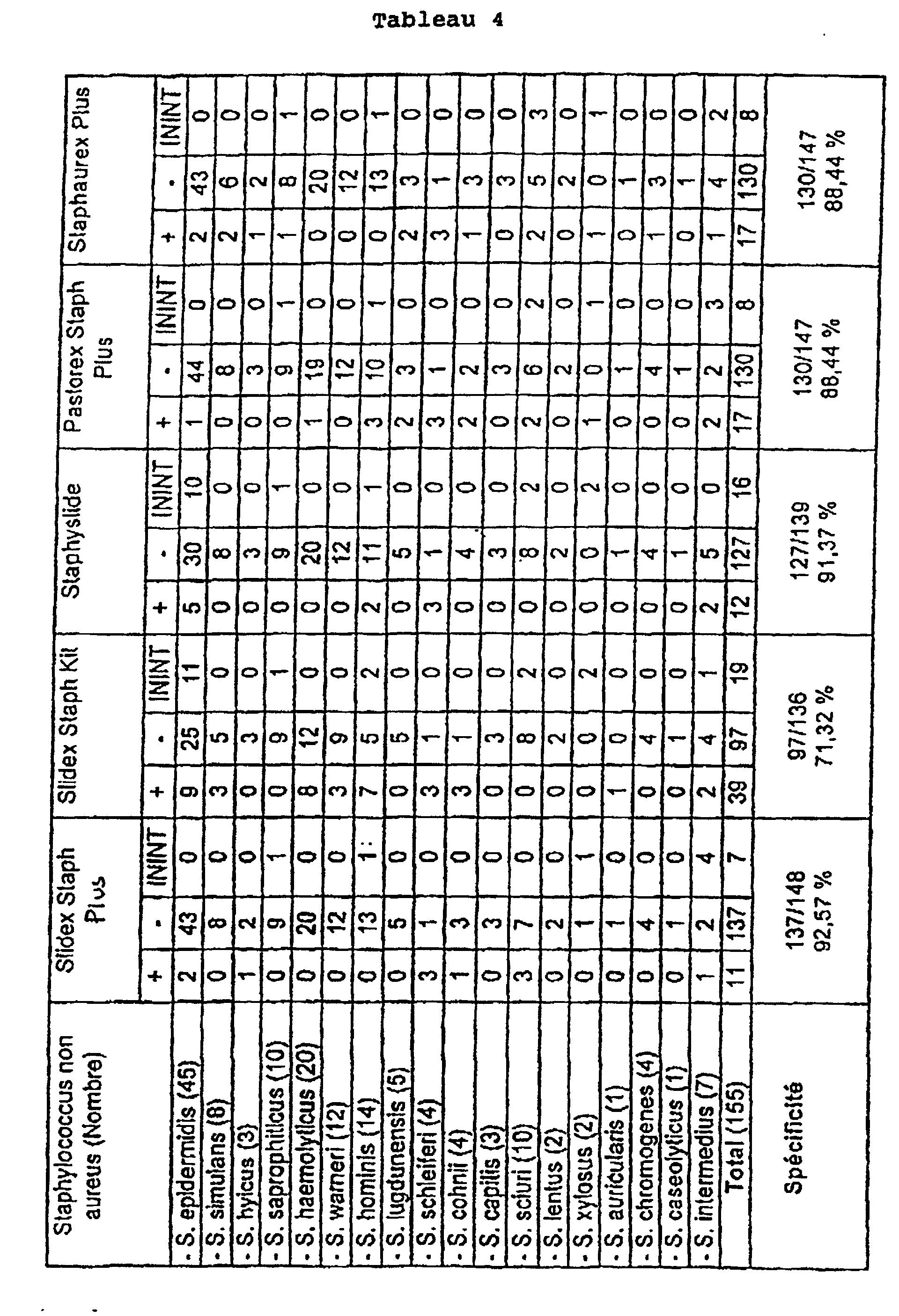
Les espèces *S. epidermidis, S. simulons, S. hyicus* peuvent posséder une capsule.
Les espèces *S. lugdunensis, S. schleiferi, S. hyicus, S. intermedius* peuvent comporter le facteur d'affinité pour le fibrinogène.
Les spécificités de PASTOREX STAPH PLUS et STAPHAUREX sont identiques (88,44 %) mais inférieures à celles de STAPHYSLIDE (91,37 %) et de SLIDEX STAPH PLUS (92,57 %).
e) Performances des réactifs
Le tableau 5 ci-dessous indique les résultats de sensibilité et de spécificité de chacun des réactifs.

**Tableau 5**

| | SENSIBILITE | | | | | SPECIFICITE |
|---|---|---|---|---|---|---|
| REACTIFS | S. aureus meti R | | | S. aureus meti S | Ensemble des souches S. aureus | |
| | Clumping Factor | | Ensemble des souches meti R | | | |
| | négatif | positif | | | | |
| Slidex staph Plus | 98.30 % | 100 % | 99.2 % | 100 % | 98.84 % | 92.57 % |
| Slidex staph | 79.67 % | 100 % | 90.36 % | 100 % | 93.0 % | 71.32 % |
| kit Staphyslide | 0 | 100 % | 52.61 % | 100 % | 65.7 % | 91.37 % |
| Pastorex | 90.6 % | 100 % | 95.56 % | 100 % | 96.81 % | 88.44 % |
| Staph Plus Staphaureux Plus | 82.9 % | 95.35 % | 89.43 % | 100 % | 92.42 % | 88.44 % |

Dans ce tableau, ainsi que dans les tableaux suivants où elles apparaissent, les mentions meti R, meti S, signifient respectivement "résistantes à la méticilline" et "sensibles à la méticilline".

Il ressort de ce tableau que la sensibilité de SLIDEX STAPH PLUS (99,2 %) est significativement supérieure aux autres réactifs ; cette supériorité est essentiellement due à une meilleure performance sur les souches *S. aureus* résistantes à la méticilline et n'ayant pas le facteur d'affinité pour le fibrinogène.

## Revendications

1. Anticorps monoclonal ou polyclonal spécifique d'un épitope commun aux souches *Staphylococcus aureus* de différents sérotypes capsulaires, **caractérisé en ce que** ledit anticorps est choisi parmi les immunoglobulines de type G, les immunoglobulines de type M, et les immunoglobulines de type A, et **en ce qu'**il reconnaît au moins le sérotype capsulaire de type 5 et le sérotype capsulaire de type 8 desdites souches de *Staphylococcus aureus.*

2. Anticorps selon la revendication 1, **caractérisé en ce qu'**il est spécifique d'un épitope commun aux souches *S. aureus* résistantes à la méticilline.

3. Anticorps monoclonal selon la revendication 1 ou 2, susceptible d'être obtenu par fusion entre la lignée de myélomes SP2/0-Ag14 (ATCC CRL 1581) et des cellules de rate de souris de l'espèce BALB/C et de la souche BALB/CBYJICO, immunisées par une souche de *Staphylococcus aureus.*

4. Anticorps monoclonal selon la revendication 1 ou 2, susceptible d'être obtenu à partir de la lignée cellulaire hybridome déposée sous le N°96021514, le 15/02/1996 auprès de l'ECACC.

5. Anticorps monoclonal selon la revendication 1 ou 2, susceptible d'être obtenu à partir de la lignée cellulaire hybridome déposée sous le N°96021513 , le 15/02/1996 auprès de l'ECACC.

6. Lignée cellulaire hybridome, telle que celle déposée sous le N°96021514, le 15/02/1996 auprès de l'ECACC, ou telle que celle déposée sous le N°96021513, le 15/02/1996 auprès de l'ECACC, ou toute autre lignée cellulaire hybridome dérivée, par exemple toute progéniture de cette lignée.

7. Réactif spécifique de détection des bactéries *Staphylococcus aureus* et notamment des souches résistantes à la méticilline, comprenant au moins un anticorps reconnaissant spécifiquement un épitope commun des différents sérotypes capsulaires des souches de *Staphylococcus aureus,* ledit anticorps étant un anticorps monoclonal ou polyclonal tel que défini selon l'une quelconque des revendications 1 à 5.

8. Réactif selon la revendication 7, **caractérisé en ce que** l'anticorps est fixé ou couplé, sur un support ou un marqueur.

9. Réactif selon la revendication 7 ou 8, comprenant en outre du fibrinogène ou un composé à base de fibrinogène, susceptible d'être reconnu par le facteur d'affinité pour le fibrinogène de *Staphylococcus aureus,* le fibrinogène ou le composé à base de fibrinogène étant éventuellement fixé ou couplé sur un support ou un marqueur.

10. Réactif selon l'une quelconque des revendications 7 à 9, comprenant en outre un ou des anticorps, reconnus par la protéine A de *Staphylococcus aureus* et étant éventuellement fixés ou couplés sur un support ou un marqueur.

11. Procédé pour détecter dans un échantillon biologique des bactéries *Staphylococcus aureus* et notamment des souches résistantes à la méticilline, selon lequel :
- on dispose d'un réactif selon l'une quelconque des revendications 7 à 10,
- on met en contact ledit échantillon avec ledit réactif,
- on observe l'obtention d'une agglutination.

## Claims

1. Monoclonal or polyclonal antibody specific for an epitope common to *Staphylococcus aureus* strains of various capsular serotypes, **characterized in that** the said antibody is selected from the type G immunoglobulins, the type M immunoglobulins and the type A immunoglobulins and **in that** it recognizes at least the type 5 capsular serotype and the type 8 capsular serotype of the said *Staphylococcus aureus* strains.

2. Antibody according to Claim 1, **characterized in that** it is specific for an epitope common to *Staphylococcus aureus* strains which are resistant to methicillin.

3. Monoclonal antibody according to Claim 1 or 2, capable of being obtained by fusion between the myeloma line SP2/0-Ag14 (ATCC CRL 1581) and spleen cells from mice of the BALB/C species and of the BALB/CBYJICO strain, immunized with a *Staphylococcus aureus* strain.

4. Monoclonal antibody according to Claim 1 or 2, capable of being obtained from the hybridoma cell line deposited under the No. 96021514, on 15/02/1996 with the ECACC.

5. Monoclonal antibody according to Claim 1 or 2, capable of being obtained from the hybridoma cell line deposited under the No. 96021513, on 15/02/1996 with the ECACC.

6. Hybridoma cell line, such as that deposited under the No. 96021514, on 15/02/1996 with the ECACC, or such as that deposited under the No. 96021513, on 15/02/1996 with the ECACC, or any other derived hybridoma cell line, for example any progeny of this line.

7. Specific reagent for the detection of *Staphylococcus aureus* bacteria and particularly methicillin-resistant strains, comprising at least one antibody specifically recognizing an epitope common to the various capsular serotypes of the *Staphylococcus aureus* strains, the said antibody being a monoclonal or polyclonal antibody as defined according to any one of Claims 1 to 5.

8. Reagent according to Claim 7, **characterized in that** the antibody is attached or coupled to a support or a marker.

9. Reagent according to Claim 7 or 8, comprising in addition fibrinogen or a compound based on fibrinogen, capable of being recognized by the *Staphylococcus aureus* affinity factor for fibrinogen, the fibrinogen or the compound based on fibrinogen being optionally attached or coupled to a support or a marker.

10. Reagent according to any one of Claims 7 to 9, comprising in addition one or more antibodies, recognized by *Staphylococcus aureus* protein A and being optionally attached or coupled to a support or a marker.

11. Process for detecting in a biological sample *Staphylococcus aureus* bacteria and particularly methicillin-resistant strains, according to which:
- a reagent according to any one of Claims 7 to 10 is made available,
- the said sample is brought into contact with the said reagent,
- the production of agglutination is observed.

## Patentansprüche

1. Monoklonaler oder polyklonaler Antikörper, der für ein gemeinsames Epitop der *Staphylococcus.* aureus-Stämme verschiedener Kapselserotypen spezifisch ist, **dadurch gekennzeichnet, daß** dieser Antikörper aus den Immunglobulinen vom Typ G, den Immunglobulinen vom Typ M und den Immunglobulinen vom Typ A ausgewählt ist, und daß dieser zumindest den Kapselserotyp 5 und den Kapselserotyp 8 der *Staphylococcus aureus*-Stämme erkennt.

2. Antikörper nach Anspruch 1, **dadurch gekennzeichnet, daß** dieser für ein gemeinsames Epitop der Meticillin-resistenten *S. aureus*-Stämme spezifisch ist.

3. Monoklonaler Antikörper nach Anspruch 1 oder 2, der durch Fusion der Myelom-Linie SP2/0-Ag14 (ATCC CRL 1581) mit Milzzellen der mit einem *Staphylococcus* aureus-Stamm immunisierten Maus vom BALB/C-Stamm und vom BALB/CYJICO-Stamm erhältlich ist.

4. Monoklonaler Antikörper nach Anspruch 1 oder 2, der von der Hybridomzellinie erhältlich ist, die am 15.02.1996 unter der Nr. 96021514 bei der ECACC hinterlegt wurde.

5. Monoklonaler Antikörper nach Anspruch 1 oder 2, der von der Hybridomzellinie erhältlich ist, die am 15.2.1996 unter der Nr. 96021513 bei der ECACC hinterlegt wurde.

6. Hybridomzellinie, wie am 15.2.1996 unter der Nr. 96021514 bei der ECACC, oder wie am 15.2.1996 unter der Nr. 96021513 bei der ECACC hinterlegt, oder jede andere abgeleitete Hybridomzellinie, bspw. jegliche Nachkommen dieser Linie.

7. Spezifisches Reagens zur Detektion von *Staphylococcus au*reus-Bakterien und insbesondere von Meticillin-resistenten Stämmen, das zumindest einen Antikörper aufweist, der spezifisch ein gemeinsames Epitop verschiedener Kapselserotypen von *Staphylococcus aureus*-Stämmen erkennt, wobei der Antikörper ein wie nach einem der Ansprüche 1 bis 5 definierter monoklonaler oder polyklonaler Antikörper ist.

8. Reagens nach Anspruch 7, **dadurch gekennzeichnet, daß** der Antikörper auf bzw. an einen Träger oder einen Marker fixiert oder gekoppelt ist.

9. Reagens nach Anspruch 7 oder 8, das außerdem Fibrinogen oder eine Verbindung auf Fibrinogenbasis aufweist, die von dem Fibrinogen-Bindefaktor von *Staphylococcus aureus* erkannt werden kann, wobei das Fibrinogen oder die Verbindung auf Fibrinogenbasis ggf. auf bzw. an einen Träger oder einen Marker fixiert oder gekoppelt ist.

10. Reagens nach einem der Ansprüche 7 bis 9, das außerdem einen oder mehrere Antikörper aufweist, die von Protein A von *Staphylococcus aureus* erkannt werden und ggf. auf bzw. an einen Träger oder einen Marker fixiert oder gekoppelt sind.

11. Verfahren zum Detektieren von Staphylococcus *aureus*-Bakterien und insbesondere von Meticillin-resistenten Stämmen in einer biologischen Probe, in dem
- ein Reagens nach einem der Ansprüche 7 bis 10 bereitgestellt wird,
- die Probe mit dem Reagens in Kontakt gebracht wird,
- der Erhalt einer Agglutination beobachtet wird.
